# EUROPÄISCHE PATENTSCHRIFT

(11) **EP 1 036 325 B1**
(45) Veröffentlichungstag und Bekanntmachung des Hinweises auf die Patenterteilung: **13.08.2008**
(21) Anmeldenummer: 99969789.9
(22) Anmeldetag: 30.09.1999
(51) Int. Cl.: G01N 33/50, G01N 33/487

(54) **VERFAHREN UND TESTBESTECK ZUR BEURTEILUNG DER OXIDATIVEN MODIFIKATION PROTEINHALTIGER STOFFE MITTELS MESSUNG IHRER ANTIRADIKALEN AKTIVITÄT**
METHOD AND TEST KIT FOR EVALUATING THE OXIDATIVE MODIFICATION OF SUBSTANCES CONTAINING PROTEIN BY MEASURING THEIR ANTIOXIDATIVE ACTIVITY
PROCEDE ET NECESSAIRE D'ANALYSE DESTINES A L'EVALUATION LA MODIFICATION OXYDANTE DE MATIERES CONTENANT DES PROTEINES PAR MESURE DE LEUR ACTIVITE ANTIRADICALE

(30) Priorität: 01.10.1998 DE 19846148
(43) Veröffentlichungstag der Anmeldung: 20.09.2000
(73) Patentinhaber: Popov, Igor, 10115 Berlin (DE); Lewin, Gudrun, 10115 Berlin (DE)
(72) Erfinder: Popov, Igor, 10115 Berlin (DE); Lewin, Gudrun, 10115 Berlin (DE)
(74) Vertreter: Wehlan, Helmut
(86) Internationale Anmeldenummer: PCT/DE1999/003234
(87) Internationale Veröffentlichungsnummer: WO 2000/019196

(56) Entgegenhaltungen:
- WO-A-91/19979
- DE-C2- 19 846 148
- US-A- 5 807 675
- AHOTUPA, M. ET AL.: "Simple methods of quantifying oxidation products and antioxidant potential of low density lipoproteins." CLINICAL BIOCHEMISTRY, Bd. 29, Nr. 2, 1996, Seiten 139-144, XP000879280
- TWIGG, J. ET AL.: "Iatrogenic DNA damage induced in human spermatozoa during sperm preparation: protective significance of seminal plasma" MOLECULAR HUMAN REPRODUCTION, Bd. 4, Nr. 5, Mai 1998 (1998-05), Seiten 439-445, XP000879291
- PACIFICI, R.E. AND DAVIES, K.J.A.: "Protein degradation as an index of oxidative stress" METHODS IN ENZYMOLOGY, Bd. 186, 1990, Seiten 485-502, XP000879263 in der Anmeldung erwähnt

## Beschreibung

Die Erfindung betrifft ein Verfahren und ein Testbesteck zur quantitativen Beurteilung der oxidativen Modifikation proteinhaltiger Stoffe bzw. des oxidativen Stresses in biologischen proteinhaltigen Proben, wie Körperflüssigkeiten, Gewebehomogenaten oder Zell-Lysaten mit Hilfe der Messung der antiradikalen Eigenschaften ihrer proteinhaltigen Komponenten.

Oxidativer Streß ist ein allgemeines Phänomen, welches in die Ätiopathogenese mehrerer Krankheiten wie Atherosklerose. Krebs, akute Entzündungen u.v.a. verwickelt ist. Als Merkmal bzw. Kriterium des Schweregrades des oxidativen Stresses wurde bis jetzt routinemäßig die Konzentration von Thiobarbitursäure (TBS)-reaktiven Spezies (Produkten der Lipidperoxidation - hauptsächlich Malondialdehyd und 4-Hydroxynonenal) bzw. konjugierten Dienen angenommen. Zu den Nachteilen dieser Methoden gehören zum Teil die relative Unspezifität wegen Interferenzen, da mehrere Substanzen mit der Testsubstanz TBS reagieren und die relative Unempfindlichkeit, da die Lipidperoxidation nicht sofort den oxidativen Streß begleitet, sondern erst nachdem die Antioxidantien verbraucht werden (FAVIER, A.: Oxidative stress: value of its demonstration in medical biology and problems posed by the choice of a marker, Ann Biol Clin (Paris), Vol. 55. 1997, p.p. 9-16).

Im Unterschied zu Lipiden reagieren Proteine sofort auf die oxidative Belastung. In den sog. Protein Degradation Assays (PACIFICI. Robert E.: DAVIES, Kelvin J.A.: Protein Degradation as an Index of Oxidative Stress. In: METHODS IN ENZYMOLOGY, Vol. 186, Part B, Eds. Packer, L. and Glazer, A.N., Academic Press, Inc.. 1990. p.p. 485-502) werden verschiedene Alterationen (vor allem der Aminosäuren) nachgewiesen. Dazu gehören die Bildung charakteristischer Produkte, Änderungen der sekundären. tertiären und quartemären Struktur, elektrischer Ladung, Faltung, Hydrophobidität. Fragmentation. kovalente inter- und intramolekulare Querverbindungen oder Erhöhung der Proteolyseempfindlichkeit. Ahotupa et al., Clinical Biochem., 29 (2) 1996, 5. 139-144 beschäftigt sich mit dem Nachweis der oxidativen Modifikation von LDL, wobei LDL von anderen hochmolekularen komponenten separiert wird.

Die Bestimmung dieser Parameter ist sehr kompliziert (teuer. langwierig, oft unspezifisch): zu den erforderlichen Meßmethoden gehören u.a. radioaktive oder fluoreszente Markierung, Gelelektrophorese. Western Blots und Immunpräzipitation.

Die Aufgabe der Erfindung besteht darin, ein neues Verfahren für die Beurteilung des oxidativen Stresses zu entwickeln. Eine weitere Aufgabe besteht darin. ein Testbesteck zu entwickeln, das zur Beurteilung des oxidativen Stresses im Organismus durch Untersuchungen von Körperflüssigkeiten, vorzugsweise Blutplasma, geeignet ist.

Die Aufgaben der Erfindung wurden durch den Gegenstand der gegenwärtigen Ansprüche gelöst.

Das Wesen der Erfindung besteht in einer Kombination bekannter und neuer Elemente, die in ihrer Gesamtheit einen synergistischen Effekt ergeben.

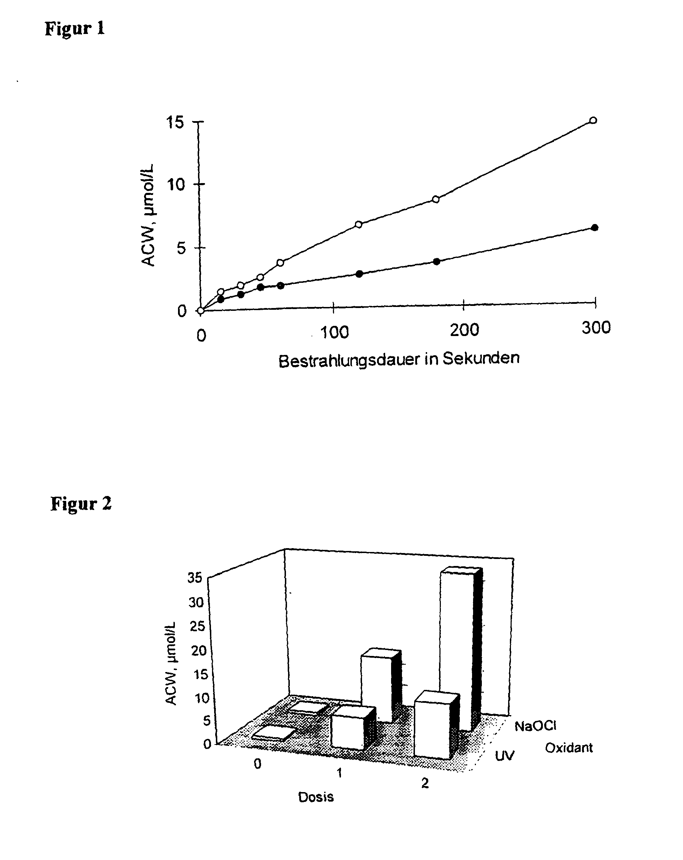

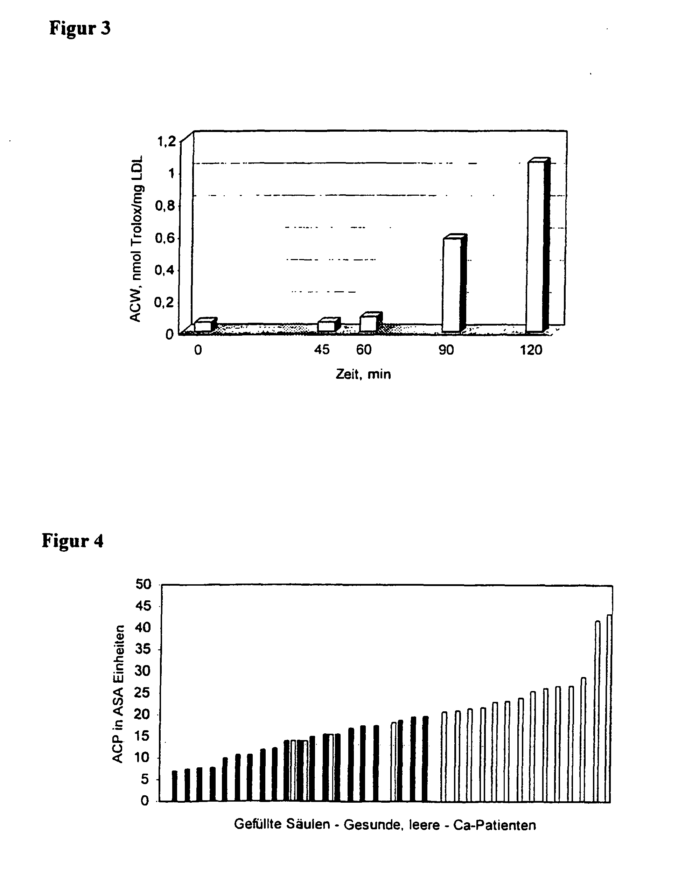

Bei den Untersuchungen zur heilenden Wirkung der ultravioletten Blutbestrahlung wurde überraschenderweise festgestellt, daß während der UV-B Bestrahlung des Blutplasmas ihre antioxidative Kapazität steigt, statt des erwarteten Abfalls. Eine vertiefte Untersuchung dieses Phänomens hat erwiesen, daß diese Erhöhung der antioxidativen Kapazität durch sieben Aminosäuren (Cystein, Histidin, Methionin, Phenylalanin. Serin, Tryptophan und Tyrosin) bedingt werden kann, da ihre antioxidative Kapazität während der Bestrahlung zustande kommt und dosisabhängig ansteigt. Ähnlich ändert sich während Oxidation auch die antioxidative Kapazität des Präparates HSA (Humanserumalbumin): Fig. 1. In der Fig. 2 sind beispielsweise die Oxidationseffekte des Histidins dargestellt. Die Effekte der Cu²⁺-induzierten Oxidation von LDL (low density lipoprotein) auf seine antioxidative Kapazität sind in der Fig. 3 dargestellt.

Diese früher nicht bekannten Eigenschaften von Blutkomponenten liefern die Voraussetzung für die Bestimmung des oxidativen Stresses in lebenden Organismen durch Untersuchung ihrer proteinhaltiger Bestandteile.

Das erfindungsgemäße Testbesteck zur Beurteilung des oxidativen Stresses im Organismus mittels Photochemolumineszenz (PCL)-Untersuchungen besteht aus 0.2 molarer Karbonatlösung und aus Luminol. Als Karbonat findet vorzugsweise Natriumkarbonat Verwendung. Zu dieser Lösung wird ein Eluat gegeben, das aus der zu untersuchenden Probe nach Durchlauf einer Entsalzungssäule und Eluierung mit PBS - Phosphate Buffer Saline - gewonnen wird.

Überraschenderweise hat sich herausgestellt, daß die im PCL-Meßsystem gemessenen Werte der antiradikalen (antioxidativen) Kapazität der aus dem Blutplasma gewonnenen Proteine nach der oxidativen Belastung sowohl durch chemische (z.B. Hypochlorit) als auch physikalische (z.B. ultraviolettes Licht) Faktoren dosisabhängig ansteigt und diese Fähigkeit zumindest 24 Stunden nach der Behandlung unverändert bleibt. D.h., es besteht die Möglichkeit, nach der Blutentnahme und Aussonderung von Proteinen den Grad der oxidativen Belastung im Organismus quantitativ einzuschätzen.

Die Auswertung der Meßergebnisse im PCL-Meßsystem kann in äquivalenten Konzentrationen einer geeigneten Kalibrationssubstanz (gewöhnlich Ascorbinsäure oder Trolox®), aber auch absolut in Sekunden der lag-Phase, des Wendepunktes (Maximum der ersten Ableitung) bzw. in prozentualer Hemmung (mit dem Integral als Auswertungsparameter) der PCL-Kurven realisiert werden.

Die folgenden Beispiele dienen der Verdeutlichung der Erfindung, ohne sie auf diese Beispiele zu beschränken.

### Ausführungsbeispiele

### Beispiel 1

0,5 ml Blutplasma werden in einer Entsalzungssäule der Firma BIO-Rad mit PBS-Lösung eluiert. Nach Durchlauf von 2,5 ml des Eluents werden 15 µl des Eluats aus dem darauf folgenden Gesamtvolumen von 2 ml im PCL-Meßsystem gemessen und die Verschiebung des Wendepunktes der registrierten Kurve im Vergleich mit dem der Kurve des Leerwertes auf der Basis einer entsprechenden Kalibrationskurve für die Umrechnung in die äquivalente Effektivität der Ascorbinsäure genutzt. Das Ergebnis - die antioxidative Kapazität der Plasmaproteine (ACP) - wird mit Berücksichtigung der Proteinkonzentration in der Probe (bestimmt z.B. mit der Biuret-Methode) in nmol Ascorbinsäure pro mg Protein berechnet und in Ascorbinsäure-Aequivalenten (ASA) dargestellt.
Definition: 1 ASA = 100 nmolAsc/mg Protein.

### Beispiel 2

Das Testbesteck für 100 Messungen besteht aus 4 Entsalzungssäulen und 6 Flaschen:
1 mal 120 ml 0,2 M Karbonatlösung mit 20 mg EDTA.
1 mal 200 ml deionisiertes Wasser.
4 mal 0,75 ml Luminollösung (3 mmol/l) in schwarzen Fläschchen (die Portionierung und die Abdunkelung sind wegen der hohen Lichtempfindlichkeit erforderlich).

Der gesamte Meßvorgang wird folgendermaßen gestaltet:
1 ml Puffer wird mit (1,5 - x) ml Wasser vermischt und x ml Probe (beim Leerwert reine PBS-Lösung) zugegeben.

Anschließend werden 25 µl der Luminollösung eingespritzt, und das Gemisch wird in das Photochemoluminometer gegeben.

Nach der Messung wird das Gerät 2mal mit deionisiertem Wasser gespült.

### Beispiel 3

Nach dem oben beschriebenen Meßprinzip werden menschliche Blutproben untersucht. Die Blutabnahme erfolgt aus der Vene cubitalis mit EDTA als Antikoagulanz. Gleich nach der Entnahme wird mittels Zentrifugation das Plasma von Zellen abgetrennt und bis zur Messung eingefroren. Die Auswertung der Meßergebnisse erfolgt nach der Verschiebung des Wendepunktes. Es wurden 20 Gesunde und 18 Patienten mit Mamma-Karzinom untersucht. Die Ergebnisse sind in der Fig. 4 dargestellt. Der Mittelwert der ACP bei Gesunden war 13,5 ASA, bei Krebs-Patienten 21.6 ASA. die Signifikanz des aus der Literatur bekannten höheren oxidativen Stresses bei Krebs-Patienten wurde nach diesem Parameter mit der Imumswahrscheinlichkeit p < 0,0005 eingeschätzt.

### Legende zu den Figuren

Figur 1: Antioxidative Kapazität (ACW) des Methionins, gemessen in 2 mmol/l-Lösung (untere kurve) und HSA 60g/l-Lösung (obere Kurve) während der Bestrahlung mit UV-Licht (254 nm) in äquivalenter Konzentration der Ascorbinsäure (Kalibrationssubstanz).
Figur 2: Antioxidative Kapazität des Histidins (2 mmol/L) unter Bedingungen der chemischen (NaOCl) und physikalischen (UV - 254 nm) Oxidation in äquivalenten Konzentration der Ascorbinsäure (Kalibrationssubstanz). Für UV: Dosis 1 = 60 sec, Dosis = 120 sec. Für NaOCl: nach 45-minütiger Inkubation mit 16 (Dosis 1) bzw. 32 (Dosis 2) mg/l NaOCl.
Figur 3: Antioxidative Kapazität von LDL in äquivalenter Konzentration des Trolox® (Kalibrationssubstanz) während der CU²⁺-induzierten Oxidation.
Figur 4: Ergebnisse der ACP-Messungen an Gesunden und Krebs-Patienten.
   1 ASA = 100nmolAsc/mg Protein.
   Mittelwerte: 13.5 und 21.6ASA; p < 0.0005

## Patentansprüche

1. Verfahren zur quantitativen Beurteilung der oxidativen Modifikation proteinhaltiger Stoffe mittels Untersuchung physiko-chemischer Proteineigenschaften, **dadurch gekennzeichnet, dass** eine inhomogene Probe vor der Messung von niedermolekularen Störstoffen (vor allem Antioxidantien) mit Hilfe einer gelchromatographischen Entsalzungssäule befreit wird und die antiradikalischen Eigenschaften der proteinhaltigen Bestandteile der Probe in einem freiradikalgenerierenden Meßsystem bestimmt werden, wobei eine höhere antiradikale Wirksamkeit dem höheren Grad der oxidativen Modifikation der Probe entspricht.

2. Verfahren nach den Ansprüchen 1, **dadurch gekennzeichnet, dass** für die Beurteilung des oxidativen Stresses im lebenden Organismus die biologische Probe aus Körperflüssigkeiten wie Blutplasma, aus Gewebehomogenaten oder Zell-Lysaten besteht.

3. Verfahren nach den Ansprüchen 1 bis 2, **dadurch gekennzeichnet, dass** für die Generation und den Nachweis von freien Radikalen das Photochemolumineszenz(PCL)-Meßsystem verwendet wird.

4. Testbesteck zur Beurteilung der oxidativen Modifikation proteinhaltiger Stoffe nach den Ansprüchen 1 bis 3, **dadurch gekennzeichnet, dass** es aus einer gelchromatographischen Entsalzungssäule, einer Luminollösung, einer Karbonatlösung und einer Phosphatpufferlösung besteht.

5. Testbesteck nach Anspruch 4, **dadurch gekennzeichnet, dass** es aus einer Entsalzungssäule, einer Luminollösung mit 3 mmol/l Luminol, 0,2 molarer Karbonatlösung mit einem pH-Wert von 10,6 und PBS-Lösung mit einem pH-Wert von 7,4 besteht.

## Claims

1. Procedure for the quantitative evaluation of the oxidative modification of protein-containing materials by means of investigation of physical-chemical protein characteristics, by the fact **characterized** that an inhomogenous sample before the measurement will be freed from low-molecular sturgeon materials (above all antioxidants) with the help of a gelchromatographic desalting column and the anti-radical characteristics of the protein-containing components of the sample will be determined in a free-radical-generating measuring system, whereby a higher anti-radical effectiveness corresponds to the higher degree of the oxidative modification of the sample.

2. Procedure according to the Claim 1, by the fact **characterized** that for the evaluation of the oxidative stress in the living organism the biological sample consists of body fluids such as blood plasma, tissue homogenate or cell lysate.

3. Procedure according to the Claims 1 to 2, by the fact **characterized** that for the generation and the proof of free radicals the measuring system photo-chemiluminescence (PCL) is used.

4. Test kit for the evaluation of the oxidative modification of protein-containing materials according to the Claims 1 to 3, by the fact **characterized** that it consists of a gelchromatographic desalting column, a luminol solution, a carbonate solution and a phosphate buffer solution.

5. Test kit according to the Claim 4, by the fact **characterized** that it consists of a desalting column, luminol solution with 3 mmol/l luminol, 0.2 molar carbonate solution with a pH value of 10,6 and PBS solution with a pH value of 7,4.

## Revendications

1. Procédé d'évaluation quantitative de la modification oxydative de substances contenant des protéines à l'aide d'une analyse des caractéristiques physico-chimiques des protéines, **caractérisé en ce qu'**un échantillon inhomogène est avant la mesure libéré d'impuretés de faible poids moléculaire (surtout des antioxydants) à l'aide d'une colonne de dessalement chromatographique sur gel et que les caractéristiques anti-radicales des éléments de l'échantillon qui contiennent des protéines sont déterminées dans un système de mesure qui génère des radicaux libres, une action plus importante anti-radicale correspondant alors à un degré plus élevé de la modification oxydative de l'échantillon.

2. Procédé selon la revendication 1, **caractérisé en ce que** l'échantillon biologique qui permet l'évaluation du stress oxydatif dans l'organisme vivant est composé de fluides corporels comme le plasma sanguin, des homogénats de tissu ou des lysats cellulaires.

3. Procédé selon l'une des revendications 1 à 2, **caractérisé en ce que** l'on utilise le système de mesure à photo-chimiluminescence pour générer les radicaux libres et déterminer leur présence.

4. Kit de test pour l'évaluation de la modification oxydative des substances contenant des protéines selon l'une des revendications 1 à 3, **caractérisé en ce qu'**il comprend une colonne de dessalement chromatographique sur gel, une solution de luminol, une solution carbonate et une solution tampon phosphate.

5. Kit de test selon la revendication 4, **caractérisé en ce qu'**il comprend une colonne de dessalement, une solution de luminol avec 3 mmol/l de luminol, une solution carbonate au 0,2 molaire avec un ph de 10,6 et une solution PBS (solution tampon phosphate) avec un ph de 7,4.
